# EUROPÄISCHE PATENTSCHRIFT

(11) **EP 2 884 948 B1**
(45) Veröffentlichungstag und Bekanntmachung des Hinweises auf die Patenterteilung: **09.11.2016**
(21) Anmeldenummer: 13747646.1
(22) Anmeldetag: 08.08.2013
(51) Int. Cl.: A61F 2/80

(54) **VERFAHREN ZUM EINSTELLEN EINES DRUCKES**
METHOD FOR ADJUSTING A PRESSURE
PROCÉDÉ DE RÉGLAGE D'UNE PRESSION

(30) Priorität: 16.08.2012 DE 102012016197
(43) Veröffentlichungstag der Anmeldung: 24.06.2015
(73) Patentinhaber: Otto Bock HealthCare GmbH, 37115 Duderstadt (DE)
(72) Erfinder: MOSLER, Lüder, 37115 Duderstadt (DE); SCHÖNEMEIER, Mark, 37077 Göttingen (DE)
(74) Vertreter: Friedrich, Andreas
(86) Internationale Anmeldenummer: PCT/EP2013/002371
(87) Internationale Veröffentlichungsnummer: WO 2014/026749

(56) Entgegenhaltungen:
- WO-A1-2008/073286
- US-A1- 2007 191 965

## Beschreibung

Die Erfindung betrifft ein Verfahren zum Einstellen eines Druckes in einem Volumen in einem Unterdruckschaftsystem.

Um Prothesensysteme, die amputierte Gliedmaßen ersetzen sollen, am Amputationsstumpf des Patienten sicher zu befestigen, sind aus dem Stand der Technik eine Reihe unterschiedlicher Möglichkeiten bekannt. Dabei kann beispielsweise über den Amputationsstumpf zunächst ein Liner gezogen werden, der beispielsweise aus einem elastischen Material, wie einem Silikon, besteht. Dieser sorgt für ein angenehmes Tragegefühl und ist aufgrund der Elastizität in der Lage, die optimale Passform für den jeweiligen Amputationsstumpf anzunehmen. An diesem Liner muss nun der eigentliche Prothesenschaft, an dem sich der weitere Prothesenaufbau befindet, angeordnet werden. Dies geschieht häufig durch einen Unterdruck, durch den das Prothesensystem am Amputationsstumpf des Patienten gehalten wird. Die Stärke des Unterdruckes, der in diesem Volumen herrscht, ist dabei sowohl ausschlaggebend für eine sichere Befestigung der Prothese am Amputationsstumpf als auch für ein angenehmes Tragegefühl der Prothese für den Patienten. Es ist daher von größter Wichtigkeit, diesen Unterdruck bzw. den in dem Volumen herrschenden Druck möglichst optimal einstellen zu können.

Wird die Prothese über einen Unterdruck am Amputationsstumpf des Patienten gehalten, spricht man von einem Unterdruckschaftsystem. Häufig wird der Unterdruck dabei zwischen dem über den Amputationsstumpf gezogenen Liner und einem Prothesenschaft erzeugt. Alternativ ist jedoch beispielsweise auch denkbar, dass der Unterdruck direkt zwischen einem Prothesenschaft oder einem anderen Bauteil des Prothesensystems und dem nackten Amputationsstumpf erzeugt wird. Dazu muss das Volumen natürlich luftdicht abgeschlossen werden, was beispielsweise über eine am oberen Ende des Prothesenschaftes angeordnete Dichtlippe aus einem dichtenden Material, beispielsweise Silikon, geschehen kann. Neben diesen beiden gängigsten Möglichkeiten, eine Prothese über einen Unterdruck am Amputationsstumpf eines Patienten zu befestigen, sind auch eine Vielzahl anderer Möglichkeiten denkbar.

Wird der Unterdruck zu stark eingestellt, ist der Druck in dem Volumen also zu gering, ist zwar eine sichere Befestigung der Prothese am Amputationsstumpf gewährleistet, es kommt jedoch insbesondere bei langem Sitzen oder anderen lange andauernden unbelasteten Zuständen zu einem unangenehmen und ggf. schmerzhaften Tragegefühl der Prothese für den Patienten. Wird hingegen ein zu geringer Unterdruck in dem Volumen erzeugt, ist der Druck in dem Volumen also zu groß, hat der Patient oftmals das Gefühl, die Prothese sei lose oder instabil. Daher sind bei modernen Prothesensystemen oftmals bereits Vakuumpumpen vorgesehen, die durch eine Fluidverbindung beispielsweise mit einem Innenraum des Prothesenschaftes verbunden sind, sodass durch die Vakuumpumpe nach dem Anlegen des Prothesensystems das Volumen zwischen dem Liner und dem Prothesensystem mit einem Unterdruck beaufschlagt werden kann.

Beim Verwenden der Prothese, beispielsweise zum Gehen, treten bei jedem Schritt Zug- und Druckphasen auf, die insbesondere die Verbindung zwischen dem Amputatiönsstumpf beziehungsweise dem über ihn gezogenen Liner und dem Prothesenschaft belasten. Das Volumen zwischen dem Amputationsstumpf oder dem Liner und dem Prothesenschaft ist dabei in der Regel nicht stabil, da es beispielsweise Gewebelagen und elastische Materialien umfasst, so dass es hier zu zwischen beiden Lagen angeordneten kompressiblen Zwischenvolumina oder sogar zu partiellem Ablösen des Liners vom Schaft kommen kann. Durch die durch die Verwendung der Prothese ausgeübten Zug- und Druckkräfte kommt es folglich zu geringfügigen Schwankungen des Volumens in dem Unterdruckschaftsystem. Da dieses Volumen im Optimalfall hermetisch abgeschlossen ist, kommt es zu Druckschwankungen im Innern des Volumens.

Aus dem Stand der Technik sind eine Reihe von Möglichkeiten bekannt, den Druck in dem Volumen zu steuern.

Aus der US 2006/0212128 A1 , die den nächstliegenden Stand der Technik darstellt, ist beispielsweise bekannt, einen oberen und einen unteren Grenzwert für den Unterdruck vorzusehen. Dabei wird jeweils die Druckdifferenz zwischen dem Druck in dem Volumen und einem das Prothesensystem umgebenden Umgebungsdruck gemessen. Diese Druckdifferenz muss zwischen den beiden festgelegten Grenzwerten liegen. Ist die Druckdifferenz zu gering, was beispielsweise durch Leckagen oder kleine Undichtigkeiten geschehen kann, wird die Pumpe aktiviert, bis die Druckdifferenz bei dem oberen Grenzwert liegt. Ähnliche Lösungen werden auch in der US 8,007,543 B2, der US 2011/0060421 A1, der US 2007/191965 A1 und der US 2010/0312361 A1 vorgeschlagen.

Insbesondere Beinprothesen sind jedoch im Gebrauch erheblichen Druckschwankungen ausgesetzt, da beim Gehen mit einer derartigen Prothese zumindest zeitweise das volle Körpergewicht des Patienten auf der Prothese lastet. Auch der Druck innerhalb des Volumens zwischen dem über den Amputationsstumpf gezogenen Liner und dem Prothesensystem ist dabei erheblichen Schwankungen unterworfen. Es ist folglich möglich, dass der Druck kurzzeitig außerhalb des durch die Grenzwerte definierten Bereichs liegt, ohne dass es zu Nachteilen kommt. Zudem ist der optimale Druck in dem Volumen abhängig von dem Bewegungszustand der Prothese. Sitzt der Patient beispielsweise für eine längere Zeit, kann der Druck erhöht werden, sodass die Druckdifferenz zwischen den im Volumen herrschenden Druck und dem die Prothese umgebenden Umgebungsdruck abgesenkt wird. Damit wird auch die Haltekraft, die durch den Unterdruck auf das Prothesensystem ausgeübt wird, verringert. Da die Prothese jedoch in diesem Zustand nicht belastet wird, kommt es nicht zu einem unbequemen Tragegefühl für den Patienten. Ein Nachpumpen des Vakuums ist in diesem Zustand nicht nötig.

Um derartige Bewegungszustände voneinander zu unterscheiden zu können, wird beispielsweise in der WO 2008/073286 A1 und der US 2011/0125291 A1 vorgeschlagen, einen Computer in das Prothesensystem zu integrieren, wobei bestimmte Charakteristika im zeitlichen Druckverlauf der einzelnen Bewegungszustände erkennt und für jeden der so erkannten Bewegungszustände separate Grenzwerte zugrunde legt, zwischen denen der gemessene Druck liegen sollte.

Damit können zwar für unterschiedliche Bewegungszustände jeweils angepasste Druckwerte verwendet werden, der Aufbau und das Steuerverfahren ist jedoch aufwendig und somit insbesondere für den Patienten kostenintensiv. Zudem ist das Verfahren fehleranfällig und wartungsintensiv. Natürlich variieren die einzelnen Druckverläufe in unterschiedlichen Bewegungszuständen, beispielsweise Stehen, Sitzen, Laufen oder Gehen von Patient zu Patient, sodass es durchaus möglich ist, dass der Computer eine Übereinstimmung des jeweils gemessenen Druckverlaufs mit den gespeicherten Vergleichsdruckverläufen nicht erkennt.

Der Erfindung liegt somit die Aufgabe zugrunde, ein Verfahren zum Einstellen des Druckes in dem Volumen in einem Unterdruckschaftsystem vorzuschlagen, das einfach auszuführen ist, verlässliche und reproduzierbare Ergebnisse liefert und zudem für den jeweiligen Bewegungszustand einen möglichst optimalen Druck liefert.

Die Erfindung löst die gestellte Aufgabe durch ein gattungsgemäßes Verfahren, das folgende Schritte aufweist:
a) Bestimmen von Druckwerten, die ein Maß für den Druck in dem Volumen sind, zu verschiedenen Zeitpunkten und Speichern der bestimmten Druckwerte,
b) Bestimmen eines Maximalwertes und eines Minimalwertes aus den gespeicherten Druckwerten,
c) Bestimmen eines Differenzwertes aus dem Maximalwert und dem Minimalwert,
d) Vergleichen des Differenzwertes mit einem vorbestimmten ersten Grenzwert und
e) Aktivieren einer Pumpe zum Absaugen eines Fluids aus dem Volumen wenn der Differenzwert größer als der vorbestimmte erste Grenzwert ist.

Die meisten Bewegungszustände einer Beinprothese, beispielsweise Gehen oder Laufen, haben einen periodisch, also zeitlich wiederkehrenden Druckverlauf innerhalb des Volumens in dem Unterdruckschaftsystem zur Folge. So wird beim Belasten der Prothese der Druck in dem Volumen steigen, sodass die Druckdifferenz zwischen dem in dem Volumen herrschenden Druck und dem das Prothesensystem umgebenden Umgebungsdruck abfällt. Ist die Prothese jedoch nicht belastet und befindet sich der Prothesenfuß in der Schwungphase, wird der Druck in dem Volumen absinken und die Druckdifferenz zwischen dem in dem Volumen herrschenden Druck und dem das Prothesensystem umgebenden Ausgangsdruck ansteigen. Der Erfindung liegt die Erkenntnis zugrunde, das unabhängig vom Bewegungszustand die Differenz zwischen dem Maximalwert und dem Minimalwert der so ermittelten Druckwerte einen vorbestimmten ersten Grenzwert nicht überschreiten sollte.

Dieser vorbestimmte erste Grenzwert kann überraschenderweise als unabhängig vom Absolutwert des in dem Volumen herrschenden Druckes angesehen werden. Es ist also für den Differenzwert gleichgültig, ob der Patient beispielsweise langsam schlendert oder schnell läuft. Solange die Differenz kleiner ist als der vorbestimmte erste Grenzwert ist, ist gewährleistet, dass die Prothese einen sicheren Halt am Amputationsstumpf aufweist und der Patient zudem ein bequemes und sichere Tragegefühl hat.

Wenn der Patient beispielsweise mit seinem Prothesensystem auf einem Stuhl sitzt, ist der Differenzwert sehr klein. Es kommt nicht zu einem sich periodisch wiederholenden Druckverlauf, sodass der in dem Volumen herrschende Druck nahezu konstant ist. Sollte das Prothesensystem über eine Leckage verfügen, durch die beispielsweise Umgebungsluft in das Volumen in dem Unterdruckschaftsystem eindringen kann, wird der Druck in dem Volumen sich immer mehr dem Umgebungsdruck annähern. Dies ist jedoch, da die Prothese nicht belastet wird, zumindest in großen Druckbereichen unproblematisch und führt nicht zu einem unbequemen oder unangenehmen Tragegefühl. Da sich die Differenz zwischen dem Maximalwert und dem Minimalwert der bestimmten Druckwerte bei diesem Vorgang nicht ändert, wird die Pumpe mit dem erfindungsgemäßen Verfahren nicht aktiviert.

Erhebt sich nun der Patient aus seiner sitzenden Position und beginnt zu gehen, kommt es zu sich periodisch wiederholenden Druckschwankungen, sodass sich die Differenz zwischen dem Maximalwert und dem Minimalwert der gespeicherten Druckwerte stark erhöht. Der Differenzwert kann dabei den vorbestimmten ersten Grenzwert durchaus überschreiten, sodass die Pumpe aktiviert wird und Fluid aus dem Volumen abgesaugt wird. Damit wird der Druck innerhalb des Volumens reduziert und somit die Druckdifferenz zum den Prothesenaufbau umgebenden Umgebungsdruck erhöht.

Je weiter der Druck innerhalb des Volumens jedoch reduziert wird, desto geringer sind die zeitlichen Schwankungen im Druckverlauf innerhalb des Volumens. Dies bedeutet, dass der Differenzwert zwischen dem Maximalwert und dem Minimalwert der gespeicherten Druckwerte sinkt, wenn die Pumpe aktiviert wird. Damit wird je nach Bewegungszustand der Prothese bzw. des die Prothese tragenden Patienten der optimale Druck eingestellt, ohne dass Bewegungszustände erkannt, Druckverlaufsmuster gespeichert oder unterschiedliche Grenzwerte für unterschiedliche Bewegungsarten gespeichert werden müssten.

In einer bevorzugten Ausführungsform werden die Druckwerte nach dem First-in-first-out-Prinzip gespeichert und es wird nur eine vorbestimmte Maximalanzahl von Druckwerten gespeichert. Ist diese Maximalzahl erreicht, wird für einen neu zu speichernden Druckwert der älteste Druckwert gelöscht. Dies entspricht dem bereits genannten First-in-first-out-Prinzip. Dadurch wird gewährleistet, dass die gespeicherten Druckwerte jeweils die aktuellsten Druckwerte sind und somit den aktuellen Prothesenzustand der Prothese am besten wiedergeben.

Vorteilhafterweise werden der Minimalwert, der Maximalwert und der Differenzwert zu verschiedenen Zeitpunkten bestimmt und die Verfahrensschritte d) und e) für jeden bestimmten Differenzwert ausgeführt. Zu unterschiedlichen Zeitpunkten, die vorteilhafterweise zeitlich möglichst nah beieinander liegen, werden die Maximalwerte, die Minimalwerte und die Differenzwerte bestimmt. Für jeden so bestimmten Differenzwert findet zudem in vorteilhafter Weise der Vergleich mit dem vorbestimmten ersten Grenzwert statt, sodass auch eine Aktivierung der Pumpe falls notwendig für jeden bestimmten Differenzwert erfolgen kann. Damit ist gewährleistet, dass zeitnah auf Änderungen im Druckverlauf reagiert werden kann, sodass fehlerhafte Druckeinstellungen in dem Volumen schnell erkannt und behoben werden können. Vorzugsweise werden wenigstens 10, besonders vorzugsweise wenigstens 50 oder höchst vorzugsweise wenigstens 100 Differenzwerte pro Sekunde bestimmt.

Vorteilhafterweise werden der Maximalwert, der Minimalwert und der Differenzwert in regelmäßigen zeitlichen Abständen, vorzugsweise nach dem Speichern jedes Druckwertes, bestimmt. Durch das regelmäßige Bestimmen des Differenzwertes kommt es zu einer besonders effizienten Überwachung des Drucks im Volumen. Natürlich ist es sinnvoll, dass zwischen zwei aufeinanderfolgenden Bestimmungen des jeweiligen Differenzwertes neue Druckwerte bestimmt und gespeichert werden. Ansonsten würde bei zwei aufeinanderfolgenden Bestimmungen des Differenzwertes das gleiche Ergebnis erzielt werden. Um eine möglichst lückenlose Überwachung des Drucks in dem Volumen zu gewährleisten und gleichzeitig eine möglichst schnelle Reaktion auf Schwankungen des Differenzwertes zu ermöglichen, ist es von Vorteil, nach jedem Abspeichern eines Druckwertes den entsprechenden Maximal- und Minimalwert und daraus den Differenzwert neu zu bestimmen und diesen mit dem vorbestimmten ersten Grenzwert zu vergleichen und gegebenenfalls die Pumpe zu aktivieren.

Als vorteilhaft hat sich herausgestellt, wenn die Pumpe deaktiviert wird, wenn der Differenzwert kleiner als der vorbestimmte erste Grenzwert ist. Damit ist gewährleistet, dass der so eingestellte Druck innerhalb des Volumens groß genug ist, um einen sicheren Halt und eine sichere Funktionsweise der Prothese am Amputationsstumpf zu gewährleisten und gleichzeitig klein genug ist, um Schwellungen, Druckstellen und andere Unbequemlichkeiten beim Tragen der Prothese für den Patienten zu vermeiden.

Bewegt sich der Patient mit einer Beinprothese, ist der tatsächliche Wert des Differenzwertes zwischen dem Maximalwert und dem Minimalwert der gespeicherten Druckwerte insbesondere abhängig von dem in dem Volumen eingestellten Druck. Je geringer der Druck im Volumen, also je größer die Differenz zwischen diesem Druck und dem den Prothesenaufbau beziehungsweise das Unterdruckschaftsystem umgebenden Umgebungsdrück, ist, desto geringer ist der Differenzwert. Bei den bisher beschriebenen Verfahren wurde der Differenzwert lediglich daraufhin überwacht, ob er größer als der vorbestimmte erste Grenzwert ist. Insbesondere für den Fall, dass sich der Patient mit der Prothese bewegt, kann der Differenzwert jedoch auch zu gering sein. Dies ist insbesondere dann der Fall, wenn der Druck in dem Volumen extrem stark abgesenkt wurde, was insbesondere zu Beeinträchtigungen im Tragekomfort führen kann. Daher wird in einer bevorzugten Ausgestaltung des Verfahrens der Druck in dem Volumen erhöht, wenn der Differenzwert kleiner als sein ein vorbestimmter zweiter Grenzwert ist. Das Erhöhen des Drucks in dem Volumen kann beispielsweise dadurch geschehen, dass die Pumpe nun in umgekehrter Richtung betrieben wird, sodass sie kein Fluid aus dem Volumen heraus-, sondern in das Volumen hineinpumpt. Alternativ dazu kann auch ein dafür vorgesehenes Ventil geöffnet werden.

In diesem Fall wird jedoch vorteilhafterweise der Differenzwert weiter beobachtet. Im bewegten Zustand der Prothese erhöht sich in diesem Fall nämlich der Differenzwert zwischen dem Maximalwert und dem Minimalwert der gespeicherten Druckwerte. Gefindet sich die Prothese jedoch nicht in Bewegung, weil der Patient beispielsweise über einen längeren Zeitraum sitzt, bleibt der Differenzwert sehr gering, sodass eine Erhöhung des Drucks in dem Volumen ohne eine weitere Überwachung des Differenzwerts dazu führen könnte, dass die Prothese vollständig vom Amputationsstumpf gelöst wird. Um dies zu vermeiden, wird vorteilhafterweise der den Differenzwert erhöhende Druck überwacht, um so feststellen zu können, ob er sich beim Erhöhen des Drucks ändert. Sollte dies nicht der Fall sein, wird die Erhöhung des Drucks beendet.

Vorteilhafterweise umfasst das Verfahren zusätzlich folgende Schritte:
f) Errechnen eines Mittelwertes aus den gespeicherten Druckwerten,
g) Ermitteln eines Vergleichsergebnisses durch Vergleichen des Mittelwerts mit einem vorbestimmten dritten Grenzwert und
h) Aktivieren der Pumpe in Abhängigkeit des Vergleichsergebnisses.

Diese Verfahrensschritte sind insbesondere für den Fall sinnvoll, dass der Patient die Prothese über einen längeren Zeitraum hinweg nicht bewegt, beispielsweise sitzt. In diesem Fall kann es durch Leckage zu einem Anstieg des Drucks innerhalb des Volumens kommen, sodass die Druckdifferenz zwischen dem Druck in dem Volumen und dem die Prothese umgebenden Umgebungsdruck abnimmt. Dies kann dazu führen, dass für den Fall, dass sich der Patient nach dem Sitzen wieder bewegt, beispielsweise aufsteht und umhergeht, ein zu geringer Unterdruck, also ein zu hoher Druck in dem Volumen befindet, sodass gegebenenfalls eine sichere Handhabung der Prothese nicht mehr gewährleistet ist. Um dies zu vermeiden, wird der Mittelwert der gespeicherten Druckwerte ausgerechnet und mit einem vorbestimmten dritten Grenzwert verglichen. In Abhängigkeit des so ermittelten Vergleichsergebnisses wird die Pumpe gesteuert.

Vorzugsweise werden die Druckwerte dabei durch Ermitteln einer Differenz zwischen einem das Prothesensystem beziehungsweise das Unterdruckschaftsystem umgebenden Umgebungsdruckes und dem in dem Volumen herrschenden Druck bestimmt und die Pumpe aktiviert, wenn der Mittelwert kleiner als der vorbestimmte dritte Grenzwert ist. Werden die Druckwerte auf diese Weise bestimmt, führt eine Leckage zu einem Ansteigen des Drucks im Inneren des Volumens in dem Unterdruckschaftsystem und somit zu einer Reduzierung des entsprechenden Druckwertes. Daher muss die Pumpe aktiviert werden, wenn der Mittelwert der gespeicherten Druckwerte den vorbestimmten dritten Grenzwert unterschreitet.

Alternativ dazu können die Druckwerte auch durch Ermitteln des Drucks in dem Volumen bestimmt werden und die Pumpe aktiviert werden, wenn der Mittelwert größer als der vorbestimmte dritte Grenzwert ist. In diesem Fall werden Absolutdrucksensoren eingesetzt, um den absoluten Druck innerhalb des Volumens zu bestimmen. Eine Differenz zum die Prothese umgebenden Umgebungsdruck würde in diesem Fall nicht ermittelt werden. Eine Erhöhung des Drucks innerhalb des Volumens hat in diesem Fall jedoch auch eine Erhöhung der Druckwerte zur Folge, sodass die Pumpe aktiviert werden muss, wenn der Mittelwert größer als der vorbestimmte dritte Grenzwerte ist.

Unabhängig von der Messmethode der einzelnen Druckwerte hat sich als vorteilhaft herausgestellt, wenn der Mittelwert ein gleitender Mittelwert ist. Dies bedeutet, dass beispielsweise immer alle gespeicherten Druckwerte zur Mittelwertbildung herangezogen werden.

Alternativ zu den bisher beschriebenen Möglichkeiten kann die Pumpe auch dann deaktiviert werden, wenn der so bestimmte Mittelwert um einen vorbestimmten Wert erhöht wurde. Da die für den Tragekomfort und die sichere Verwendung der Prothese maßgebliche Größe jedoch der Differenzwert ist, ist die Deaktivierung der Prothese für den Fall, dass der Differenzwert kleiner als der vorbestimmte Grenzwert ist, dieser Möglichkeit vorzuziehen. Natürlich ist auch Kombination beider Kriterien denkbar.

Eine Vakuumpumpe, die an einem Prothesensystem angeordnet ist, verfügt über eine bauartbedingte maximale Druckdifferenz, die sie zwischen dem Volumen in dem Unterdruckschaftsystem und dem Umgebungsdruck herstellen kann. Insbesondere für den Fall, dass die Druckwerte als Differenzdruckwert ermittelt werden, kann daher der Mittelwert auch dahingehend überwacht werden, ob dieser von der Pumpe bauartbedingt vorgegebene maximale Druck nahezu erreicht wird. Sollte dies der Fall sein, kein anderes Kriterium ein Abschalten der Pumpe zur Folge haben, könnte auch allein aus diesem Grund die Pumpe abgeschaltet werden, um eine Überlastung der Pumpe zu vermeiden. Eine weitere Absenkung des Drucks innerhalb des Volumens ist für die jeweilige Pumpe bauartbedingt nicht möglich, sodass eine weitere Absenkung des Druckes nicht erreicht werden kann. Um ein Überlasten der Pumpe zu vermeiden, sollte diese in diesem Fall abgeschaltet werden.

Anstelle eines vorbestimmten von der Pumpe Bauart bedingt erreichbaren maximalen Drucks könnte die Änderung des Mittelwertes während des Pumpens betrachtet werden. Bei aktivierter Pumpe sollte der Mittelwert des ermittelten Drucks sich spürbar verändern. Ist dies nicht mehr der Fall, ist dies ein deutliches Anzeichen dafür, dass die Pumpe an ihre Leistungsgrenze gestoßen ist und ein weiteres Abpumpen keinen nennenswerten Effekt mehr aufweist. Um Energie zu sparen und eine unnötige Geräuschbelastung für den Patienten zu vermeiden, sollte die Pumpe auch in diesem Fall abgeschaltet werden. Alle diese Möglichkeiten können durch die Verarbeitung der bestimmten Mittelwerte und eine entsprechende Steuerung, die beispielsweise in dem Prothesensystem angeordnet sein kann, erreicht und gegebenenfalls miteinander kombiniert werden. Mit Hilfe einer Zeichnung wird nachfolgend ein Ausführungsbeispiel der vorliegenden Erfindung näher erläutert. Es zeigt:
- Fig. 1: - ein schematisches Flussdiagramm eines Verfahrens gemäß einem ersten Ausführungsbeispiel der vorliegenden Erfindung,
- Fig. 2 und 3: - die Verläufe unterschiedlicher Messwerte während der Durchführung des Verfahrens gemäß einem weiteren Ausführungsbeispiel der vorliegenden Erfindung,
- Fig. 4: - ein Druckverlauf aus einer Steuerung gemäß dem Stand der Technik im Vergleich mit einer Steuerung gemäß einem Ausführungsbeispiel der vorliegenden Erfindung,
- Fig. 5: - die schematische Schnittdarstellung durch einen Ausschnitt eines an einem Amputationsstumpf angeordneten Prothesensystems und
- Fig. 6: - die Darstellung aus Figur 5 nach dem Erzeugen des Unterdrucks.

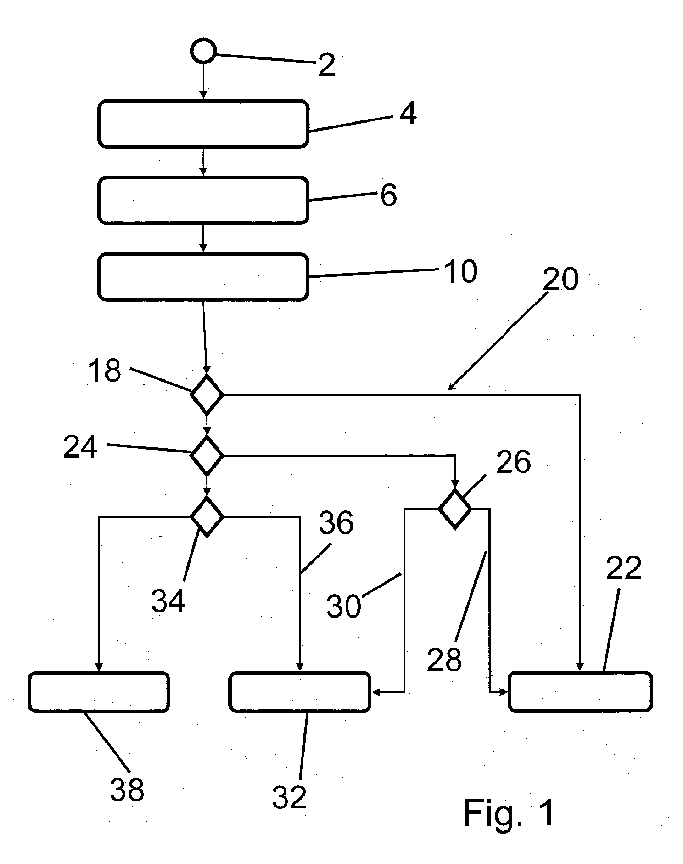

Figur 1 zeigt den schematischen Ablauf eines Verfahrens gemäß einem ersten Ausführungsbeispiel der vorliegenden Erfindung in Form eines Flussdiagramms. Das Verfahren beginnt bei einem Zeitpunkt 2, der als Auslösezeitpunkt, Timer Event oder Trigger verwendet wird. Danach erfolgt das Bestimmen des jeweiligen Druckwertes 4, der ein Maß für den Druck in dem Volumen ist. Dies kann beispielsweise durch ein Absolutdrucksensor oder einen Differenzdrucksensor geschehen, wobei ein Differenzdrucksensor die Druckdifferenz zwischen einem die Prothese umgebenden Umgebungsdruck und dem Druck im Volumen bestimmt. Der so bestimmt Druckwert wird zudem in einem Speicherelement gespeichert. Sofern in dem Speicherelement bereits eine vorbestimmte Maximalanzahl von Druckwerten gespeichert ist, wird vorzugsweise der älteste Druckwert aus dem Speicher entfernt. Dies entspricht dem so genannten First-in-first-out-Prinzip, das vorzugsweise verwendet wird.

Anschließend wird in einer Mittelwertbildung 6 ein Mittelwert 8 aus allen gespeicherten Druckwerten bestimmt. Dies kann ein arithmetisches Mittel oder ein bestimmte Umstände berücksichtigendes gewichtetes Mittel sein. Gewichtete Mittel werden insbesondere dann vorteilhafterweise eingesetzt, wenn die einzelnen Druckwerte nicht zu zeitlich äquidistanten Messpunkten aufgenommen wurden. Den unterschiedlich langen zeitlichen Intervallen zwischen den einzelnen Druckwerten kann durch die Bildung eines gewichteten Mittelwertes Rechnung getragen werden. Aus der Differenz des Maximalwerts 12 und des Minimalwerts 14 wird so ein Differenzwert 16 bestimmt. Der Mittelwert 8 und der Differenzwert 16 werden im weiteren Verlauf des Verfahrens zur Steuerung der Pumpe, die die Einstellung des Drucks in dem Volumen zuständig ist, verwendet.

Dazu wird in einem ersten Vergleich 18 bei dem in Figur 1 gezeigten Ausführungsbeispiel der Mittelwert 8 mit einem vorbestimmten dritten Grenzwert verglichen. Für den Fall, dass die Druckwerte durch Differenzdrucksensoren aufgenommen werden, bedeutet eine Verringerung des Druckwertes einen Anstieg des Drucks innerhalb des Volumens, da die Differenz zwischen dem Druck in dem Volumen und dem die Prothese umgebenden Umgebungsdruck abnimmt. Wird die Differenz zu gering, muss die Pumpe aktiviert werden, um einen ausreichend starken Unterdruck in dem Volumen zwischen dem über dem Amputationsstumpf gezogenen Liner und dem Prothesensystem hervorrufen zu können.

Im ersten Vergleich 18 wird folglich der Mittelwert 8 mit dem vorbestimmten dritten Grenzwert verglichen. Ist der Mittelwert 8 im gezeigten Auführungsbeispiel geringer als der dritte Grenzwert, führt das zu einer Pumpenaktivierung 22, was durch einen ersten Verlaufpfeil 20 dargestellt ist. Ist der Mittelwert 8 größer als der vorbestimmte dritte Grenzwert, wird in einem zweiten Vergleich 24 der Differenzwert 16 mit dem vorbestimmten ersten Grenzwert verglichen. Für den Fall, dass der Differenzwert 16 größer als der vorbestimmte erste Grenzwert ist, wird in der Prüfung 26 ermittelt, ob der Mittelwert 8 bereits den für die Pumpe bauartbedingt möglichen Grenzwert erreicht hat. Ist dies nicht der Fall, führt der Pfeil 28 ebenfalls zur Pumpenaktivierung 22. Dadurch wird der Druck in dem Volumen zwischen Liner und Prothesenelement verringert, sodass, da es sich vorliegend um Differenzdruckmessungen handelt, der Mittelwert 8 aus den gespeicherten Druckwerten ansteigt. Dies hat eine Verringerung des Differenzwertes 16 zur Folge.

Ist hingegen der Mittelwert 8 bereits am Leistungsmaximum für die vorliegende Pumpe angelangt, ist eine weitere Erhöhung des Mittelwertes 8 und damit verbunden eine Absenkung des Drucks innerhalb des Volumens mit der verwendeten Pumpe nicht mehr möglich, sodass das Verfahren dem Pfeil 30 folgt, der dazu führt, dass die Pumpe nicht aktiviert wird, was durch die Box 32 dargestellt ist.

Für den Fall, dass im zweiten Vergleich 24, bei dem der Differenzwert 16 mit vorbestimmten ersten Grenzwert verglichen wird, erkannt wird, dass der Differenzwert 16 kleiner als der vorbestimmte erste Grenzwert, wird in der Prüfung 34 ermittelt, ob der Maximalwert 12 dem Minimalwert 14 entspricht. Ist dies nicht der Fall, führt der Pfeil 36 zur Box 32, sodass die Pumpe nicht aktiviert wird. Entspricht jedoch der Minimalwert 14 dem Maximalwert 12, ist der Differenzwert im Rahmen der Messungenauigkeit nahezu Null. Dann kann es zu einer Druckerhöhung 38 kommen, wodurch der Druck in dem Volumen erhöht und somit der Mittelwert 8 gesenkt wird. Durch dieses Verfahren wird in jeglichem Bewegungszustand der Prothese ein optimaler Druck im Volumen in dem Unterdruckschaftsystem erreicht.

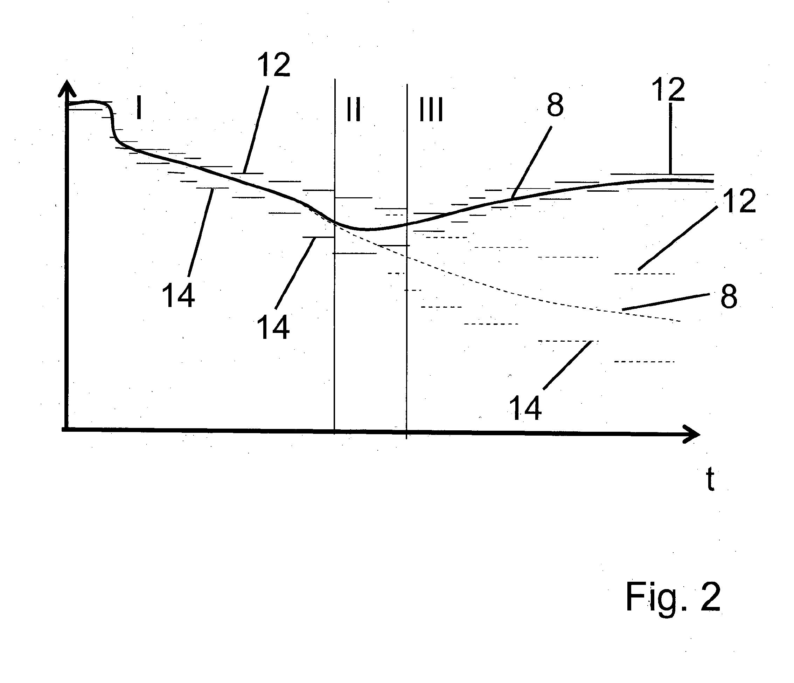

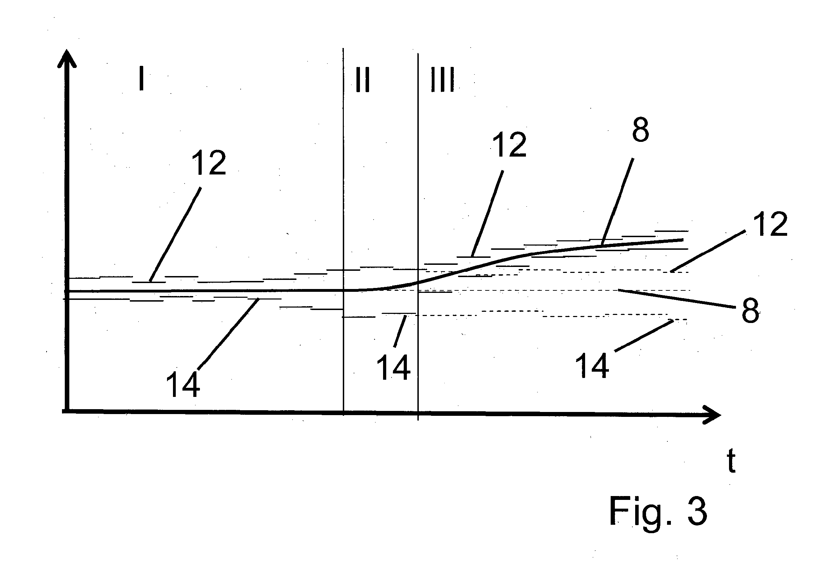

In den Figuren 2 und 3 ist in Form einer durchgezogenen Linie jeweils der Mittelwert 8 als Funktion der Zeit t dargestellt. Oberhalb des jeweiligen Mittelwertes 8 ist in Form dünner durchgezogener Striche der Maximalwert und unterhalb des Mittelwertes 8 der Minimalwert 14 dargestellt. Eine Erhöhung des Mittelwertes 8 bedeutet dabei jeweils einen stärkeren Unterdruck in dem Volumen in dem Unterdruckschaftsystem und somit einen festen Halt der Prothese.

Man erkennt, dass die Diagramme in den Figuren 2 und 3 in drei Bereiche I, II und III aufgeteilt sind.

In den Bereichen I und III ist jeweils die Differenz zwischen dem Minimalwert 14 und dem Maximalwert 12, die dem Differenzwert 16 entspricht, kleiner als der vorbestimmte erste Grenzwert, sodass in diesen Bereichen keine Pumpenaktivierung stattfindet. Im Bereich II in den Figuren 2 und 3 ist der Differenzwert 16 größer als der vorbestimmte erste Grenzwert, sodass in dem Bereich II jeweils eine Aktivierung der Pumpe erfolgt. Dies hat eine Erhöhung des Mittelwertes 8 und darin eine Reduzierung des Differenzwertes 16 bei größeren Zeiten t zur Folge.

In den in Figuren 2 und 3 gezeigten Ausführungsbeispielen der vorliegenden Erfindung wird die Pumpe jedoch nicht deaktiviert, sobald der Differenzwert 16 kleiner ist als der vorbestimmte Grenzwert. Wäre dies der Fall, dürfte sich der Mittelwert 8 in dem jeweiligen Bereich III nicht weiter erhöhen. Vorliegend ist ein anderes Kriterium zur Deaktivierung der Pumpe gewählt worden.

Man erkennt insbesondere in Figur 2, dass es im Bereich I zu einer teilweisen deutlichen Absenkung des Mittelwertes 8 kommt. Dies kann beispielsweise durch Leckage geschehen, durch die ein Fluid in das Volumen eindringen kann. Dies hat eine Reduzierung des Mittelwertes 8 zur Folge, woraus bereits erkannt werden kann, dass der Mittelwert und damit auch die Druckwerte in Form von Differenzdruckwerten vorliegen, die die Differenz zwischen dem die Prothese umgebenden Umgebungsdruck und dem Druck in dem Volumen darstellen. Eine Reduzierung dieses Mittelwertes 8 bedeutet folglich eine Erhöhung des Drucks in dem Volumen.

Sowohl in Figur 2 als auch in Figur 3 sind in Form von gestrichelten Linien sowohl der Mittelwert 8 als auch der Minimalwert 14 und der Maximalwert 12 so dargestellt, wie sie sich ohne eine Pumpenaktivierung entwickeln würden. Man erkennt, dass in beiden Figuren der Mittelwert 8 mehr oder weniger stark abfällt und der jeweilige Differenzwert 16 als Differenz zwischen dem Maximalwert 12 und dem Minimalwert 14 in Abhängigkeit von der Zeit t stark zunimmt. Dies hätte unbequemes und unsicheres Tragegefühl zur Folge, da der Patient das Gefühl hätte, die Prothese wäre lose, instabil und nicht guten Gewissens belastbar.

Durch das vorliegende Verfahren wird eine Drucksteuerung für das Volumen zwischen dem Liner und dem Prothesenelement erreicht, die mit wenigen Messwerten auskommt, sicher reproduzierbar funktioniert und zudem konstruktiv und steuerungstechnisch einfach umsetzbar ist.

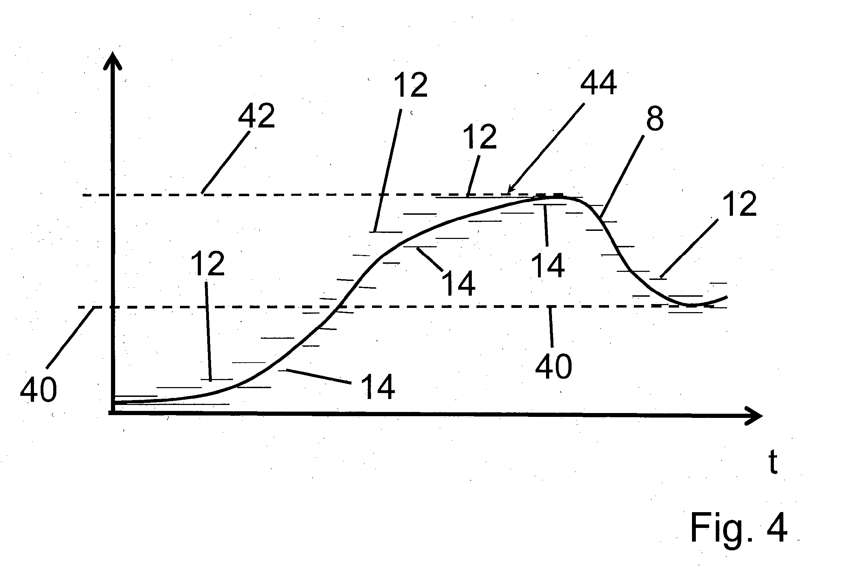

Auch Figur 4 zeigt den Mittelwert 8 als Funktion der Zeit t. Dabei entspricht der Mittelwert 8 in dem in Figur 4 gezeigten Verlauf jedoch einem Druck, der durch eine Steuerung gemäß dem Stand der Technik gesteuert wird. Dabei wird eine Pumpe so gesteuert, dass der Mittelwert 8 immer zwischen einem vorher festgesetzten Mindestwert 40 und einem vorher festgesetzten Höchstwert 42 liegt. Im linken Bereich befindet sich der Mittelwert 8 deutlich unterhalb des Mindestwertes 40, so dass eine Pumpe aktiviert wird. Dadurch wird ein Fluid aus dem Volumen entfernt, so dass es zu einem Unterdruck kommt, was durch eindeutiges Ansteigen des Mittelwertes 8 zu erkennen ist. Die Pumpe wird erst dann deaktiviert, wenn der Mittelwert 8 den Höchstwert 42 erreicht. Danach kommt es zu einem Abfall des Mittelwertes 8, was beispielsweise durch Leckagen geschehen kann. Diese sind in Figur 4 jedoch stark überzeichnet dargestellt. Die Verringerung des Unterdruckes, die mit der Verringerung des Mittelwertes 8 einhergeht, kann sich im realen Fall über mehrere Stunden hinziehen und hängt unter anderem vom Belastungszustand der Prothese ab.

Sobald der Mittelwert 8 im rechten Bereich der Figur 4 den vorbestimmen Mindestwert 40 wieder erreicht, wird die Pumpe wieder aktiviert, so dass der Mittelwert 8 wieder ansteigt.

In Figur 4 sind zudem, wie in den Figuren 2 und 3, die Maximalwerte 12 und die Minimalwerte 14 dargestellt. Man erkennt, dass der Differenzwert 16 zwischen Maximalwert 12 und Minimalwert 14, insbesondere im linken Bereich der Figur 4, in dem der Mittelwert 8 ansteigt, relativ groß ist, so dass auch durch ein Verfahren gemäß einem Ausführungsbeispiel der vorliegenden Erfindung die Pumpe aktiviert worden wäre, um einen stärkeren Unterdruck in dem Volumen in dem Unterdruckschaftsystem zu erreichen. Insbesondere in einem Bereich 44, der kurz vor dem Erreichen des vorbestimmten Höchstwertes 42 durch den Mittelwert 8 liegt, ist der Differenzwert 16 zwischen dem Maximalwert 12 und dem Minimalwert 14 jedoch relativ gering, so dass es in diesem Bereich durch ein Steuerungsverfahren gemäß einem Ausführungsbeispiel der vorliegenden Erfindung bereits zu einem Abschalten der Pumpe gekommen wäre. Dies hätte folglich eine geringere und insbesondere zeitlich kürzere Geräuschbelastung durch die Pumpe für den Patienten zur Folge gehabt. Zudem wäre ein nicht so starker Unterdruck in dem Volumen erzeugt worden, der jedoch für den vorherrschenden Belastungszustand der Prothese ausreichend gewesen wäre.

Im Bereich des Abfalls des Mittelwertes 8 in Figur 4 ist deutlich zu erkennen, dass der Differenzwert 16 zwischen dem Maximalwert 12 und dem Minimalwert 14 stark ansteigt. In diesem Bereich wäre durch ein Verfahren gemäß einem Ausführungsbeispiel der vorliegenden Erfindung die Pumpe wieder aktiviert worden, so dass ein derartig starker Abfall des Unterdrucks, wie er in Figur 4 dargestellt ist, vermieden worden wäre. Für den Patienten bedeutete dies deutlich geringere Schwankungen des eingestellten Unterdrucks und damit auch deutlich geringere Schwankungen der auf seinen Amputationsstumpf wirkenden Kräfte. Dadurch wird der Tragekomfort der Prothese deutlich erhöht.

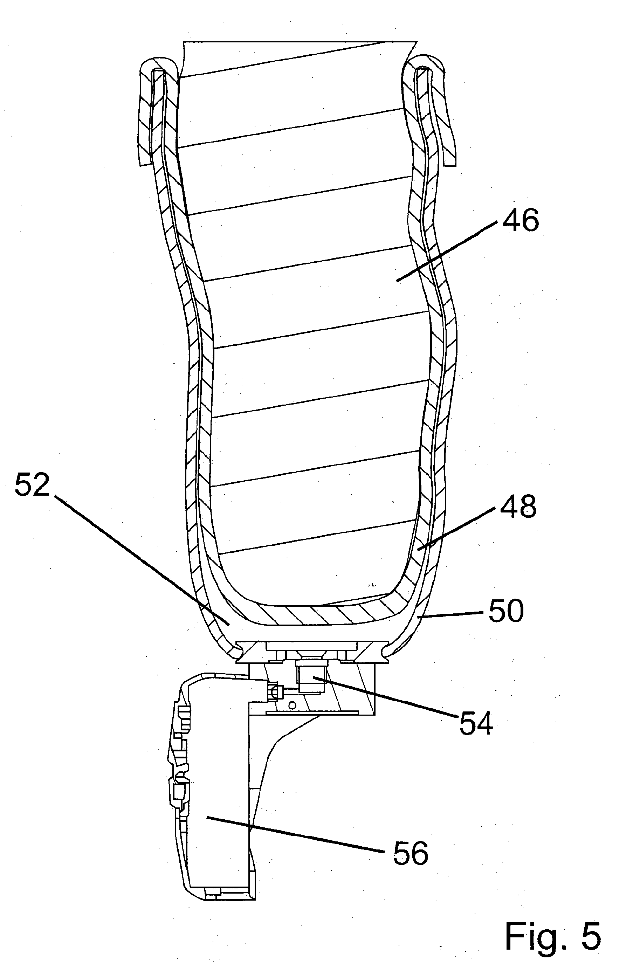

Figur 5 zeigt einen Ausschnitt einer schematischen Schnittdarstellung. Man erkennt einen Amputationsstumpf 46, über den ein Liner 48 gezogen wurde. Dieser ist in einem Prothesenschaft 50 angeordnet, wobei sich zwischen dem Liner 48 und dem Prothesenschaft 50 ein Volumen 52 befindet.

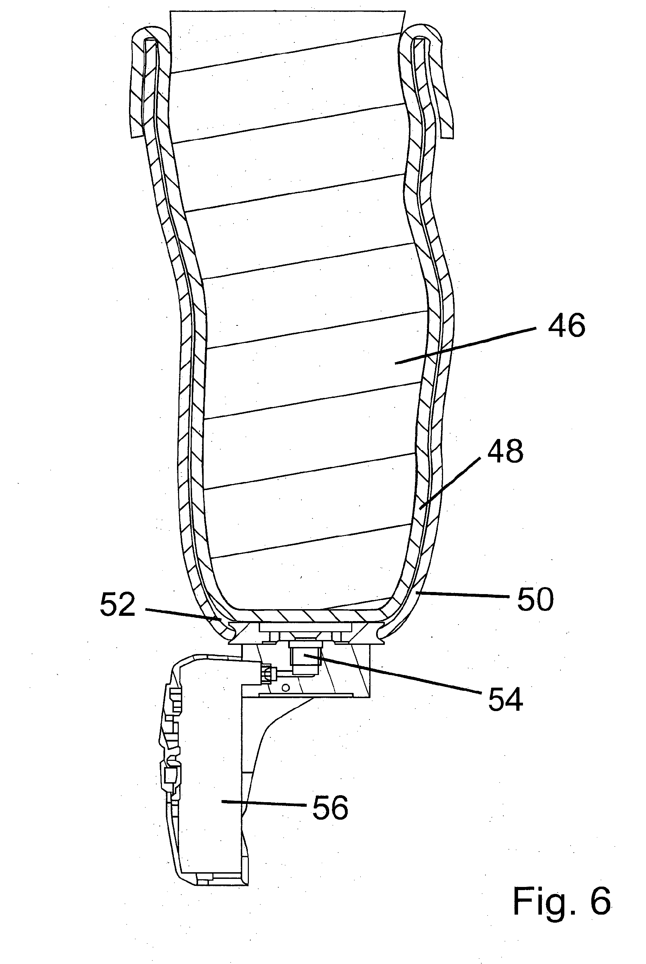

Dieses Volumen 52 ist über eine Fluidverbindung 54 mit einer Pumpe 56 verbunden, durch die ein Unterdruck in dem Volumen 52 herstellbar ist. Dies ist in Figur 6 dargestellt. Nachdem die Pumpe 56 aktiviert ist, wird in dem Volumen 52 ein Unterdruck hergestellt, wodurch der Amputationsstumpf 46 und der darüber gezogene Liner 48 in den Prothesenschaft 50 hineingezogen werden und sich der Form des Prothesenschaftes anpassen. Man erkennt, dass das Volumen 52 nur noch eine minimale Größe aufweist. Durch den in diesem Volumen 52 herrschenden Unterdruck wird der Liner 48 mit dem darin befindlichen Amputationsstumpf 46 im Prothesenschaft 50 gehalten.

**Bezugszeichenliste**

| | | | |
|---|---|---|---|
| I, II, III | Bereich | 56 | Pumpe |
| t | Zeit | | |
| 2 | Zeitpunkt | | |
| 4 | Bestimmen des Druckwertes | | |
| 6 | Mittelwertbildung | | |
| 8 | Mittelwert | | |
| 10 | Extrema-Auswertung | | |
| 12 | Maximalwert | | |
| 14 | Minimalwert | | |
| 16 | Differenzwert | | |
| 18 | erster Vergleich | | |
| 20 | Verlaufspfeil | | |
| 22 | Pumpenaktivierung | | |
| 24 | zweiter Vergleich | | |
| 26 | Prüfung | | |
| 28 | Pfeil | | |
| 30 | Pfeil | | |
| 32 | Box | | |
| 34 | Prüfung | | |
| 36 | Pfeil | | |
| 38 | Druckerhöhung | | |
| 40 | Mindestwert | | |
| 42 | Höchstwert | | |
| 44 | Bereich | | |
| 46 | Amputationsstumpf | | |
| 48 | Liner | | |
| 50 | Prothesenschaft | | |
| 52 | Volumen | | |
| 54 | Fluidverbindung | | |

## Patentansprüche

1. Verfahren zum Einstellen eines Druckes in einem Volumen (52) in einem Unterdruckschaftsystem, wobei das Verfahren folgende Schritte aufweist:
a) Bestimmen von Druckwerten (4), die ein Maß für den Druck in dem Volumen (52) sind, zu verschiedenen Zeitpunkten und Speichern der bestimmten Druckwerte,
b) Bestimmen eines Maximalwertes (12) und eines Minimalwertes (14) aus den gespeicherten Druckwerten,
c) Bestimmen eines Differenzwertes (16) aus dem Maximalwert (12) und dem Minimalwert (14),
d) Vergleichen des Differenzwertes (16) mit einem vorbestimmten ersten Grenzwert und
e) Aktivieren einer Pumpe (56) zum Absaugen eines Fluids aus dem Volumen (52) wenn der Differenzwert (16) größer als der vorbestimmte erste Grenzwert ist.

2. Verfahren nach Anspruch 1, **dadurch gekennzeichnet, dass** die Druckwerte nach dem First-In-First-Out-Prinzip gespeichert werden und nur eine vorbestimmte Maximalanzahl von Druckwerten gespeichert wird.

3. Verfahren nach Anspruch 1 oder 2, **dadurch gekennzeichnet, dass** der Maximalwert (12), der Minimalwert (14) und der Differenzwert (16) zu verschiedenen Zeitpunkten bestimmt werden und das die Verfahrensschritte d) und e) für jeden bestimmten Differenzwert ausgeführt werden.

4. Verfahren nach Anspruch 3, **dadurch gekennzeichnet, dass** der Maximalwert (12), der Minimalwert (14) und der Differenzwert (16) in regelmäßigen zeitlichen Abständen, vorzugsweise nach dem Speichern jedes Druckwertes bestimmt werden.

5. Verfahren nach einem der vorstehenden Ansprüche, **dadurch kennzeichnet, dass** die Pumpe (56) deaktiviert wird, wenn der Differenzwert (16) kleiner als der vorbestimmte erste Grenzwert ist.

6. Verfahren nach einem der vorstehenden Ansprüche, **dadurch gekennzeichnet, dass** der Druck in dem Volumen (52) erhöht wird, wenn der Differenzwert (16) kleiner als ein vorbestimmter zweiter Grenzwert ist.

7. Verfahren nach einem der vorstehenden Ansprüche, **gekennzeichnet durch** die zusätzlichen Schritte:
f) Errechnen eines Mittelwertes (8) aus den gespeicherten Druckwerten,
g) Ermitteln eines Vergleichsergebnisses **durch** Vergleichen des Mittelwerts (8) mit einem vorbestimmten dritten Grenzwert und
h) Aktivieren der Pumpe (56) in Abhängigkeit des Vergleichsergebnisses.

8. Verfahren nach Anspruch 7, **dadurch gekennzeichnet, dass** die Druckwerte durch Ermitteln einer Differenz zwischen einem das Unterdruckschaftsystem umgebenden Umgebungsdruck und dem im Volumen (52) herrschenden Druck bestimmt werden und die Pumpe (56) aktiviert wird, wenn der Mittelwert (8) kleiner als der vorbestimmte dritte Grenzwert ist.

9. Verfahren nach Anspruch **7, dadurch gekennzeichnet, dass** die Druckwerte durch Ermitteln des Druckes in dem Volumen (52) bestimmt werden und die Pumpe (56) aktiviert wird, wenn der Mittelwert (8) größer als der vorbestimmte dritte Grenzwert ist.

10. Verfahren nach Anspruch 7, 8 oder 9, **dadurch gekennzeichnet, dass** der Mittelwert (8) ein gleitender Mittelwert ist.

## Claims

1. A method for adjusting a pressure in a volume (52) in a negative-pressure socket system, said method comprising the following steps:
a) determining pressure values (4), which are a measure of the pressure in the volume (52), at different moments, and storing the pressure values that have been determined,
b) determining a maximum value (12) and a minimum value (14) from the stored pressure values,
c) determining a difference value (16) from the maximum value (12) and the minimum value (14),
d) comparing the difference value (16) with a predetermined first limit value, and
e) activating a pump (56) in order to aspirate a fluid from the volume (52) if the difference value (16) is greater than the predetermined first limit value.

2. The method as claimed in claim 1, **characterized in that** the pressure values are stored according to the first-in first-out principle, and only a predetermined maximum number of pressure values is stored.

3. The method as claimed in claim 1 or 2, **characterized in that** the maximum value (12), the minimum value (14) and the difference value (16) are determined at different moments, and **in that** method steps d) and e) are performed for each determined difference value.

4. The method as claimed in claim 3, **characterized in that** the maximum value (12), the minimum value (14) and the difference value (16) are determined at regular time intervals, preferably after the storage of each pressure value.

5. The method as claimed in one of the preceding claims, **characterized in that** the pump (56) is deactivated if the difference value (16) is less than the predetermined first limit value.

6. The method as claimed in one of the preceding claims, **characterized in that** the pressure in the volume (52) is increased if the difference value (16) is less than a predetermined second limit value.

7. The method as claimed in one of the preceding claims, **characterized by** the additional steps of:
f) calculating an average value (8) from the stored pressure values,
g) establishing a comparison result by comparing the average value (8) with a predetermined third limit value, and
h) activating the pump (56) in accordance with the comparison result.

8. The method as claimed in claim 7, **characterized in that** the pressure values are determined by establishing a difference between an atmospheric pressure surrounding the negative-pressure socket system and the pressure prevailing in the volume (52), and the pump (56) is activated if the average value (8) is less than the predetermined third limit value.

9. The method as claimed in claim 7, **characterized in that** the pressure values are determined by detecting the pressure in the volume (52), and the pump (56) is activated if the average value (8) is greater than the predetermined third limit value.

10. The method as claimed in claim 7, 8 or 9, **characterized in that** the average value (8) is a moving average.

## Revendications

1. Procédé pour le réglage d'une pression dans un volume (52) dans un système d'emboîture en dépression, ledit procédé comprenant les étapes suivantes :
a) on détermine des valeurs de pression (4) qui sont une mesure pour la pression dans le volume (52) à différents instants et on mémorise les valeurs de pression déterminées,
b) on détermine une valeur maximum (12) et une valeur minimum (14) à partir des valeurs de pression mémorisées,
c) on détermine une valeur différentielle (16) à partir de la valeur maximum (12) et de la valeur minimum (14),
d) on compare la valeur différentielle (16) avec une première valeur limite prédéterminée, et
e) on active une pompe (56) pour aspirer un fluide hors du volume (52) quand la valeur différentielle (16) est plus grande que la première valeur limite prédéterminée.

2. Procédé selon la revendication 1, **caractérisé en ce que** les valeurs de pression sont mémorisées d'après le principe "première-entrée-première-sortie" (First-In-First-Out) et on mémorise uniquement un nombre maximum prédéterminé de valeurs de pression.

3. Procédé selon la revendication 1 ou 2, **caractérisé en ce que** la valeur maximum (12), la valeur minimum (14) et la valeur différentielle (16) sont déterminées à différents instants, et **en ce que** les étapes d) et e) du procédé sont exécutées pour chaque valeur différentielle déterminée.

4. Procédé selon la revendication 3, **caractérisé en ce que** la valeur maximum (12), la valeur minimum (14) et la valeur différentielle (16) sont déterminées à des intervalles temporels réguliers, de préférence après la mémorisation de chaque valeur de pression.

5. Procédé selon l'une des revendications précédentes, **caractérisé en ce que** la pompe (56) est désactivée quand la valeur différentielle (16) est plus petite que la première valeur limite prédéterminée.

6. Procédé selon l'une des revendications précédentes, **caractérisé en ce que** la pression est augmentée dans le volume (52) quand la valeur différentielle (16) est plus petite qu'une seconde valeur limite prédéterminée.

7. Procédé selon l'une des revendications précédentes, **caractérisé par** les étapes supplémentaires suivantes :
f) on calcule une valeur moyenne (8) à partir des valeurs de pression mémorisée,
g) on détermine un résultat de comparaison par comparaison de la valeur moyenne (8) avec une troisième valeur limite prédéterminée, et
h) on active la pompe (56) en fonction du résultat de comparaison.

8. Procédé selon la revendication 7, **caractérisé en ce que** les valeurs de pression sont déterminées par détermination d'une différence entre une pression environnante qui entoure le système d'emboîture en dépression et la pression régnant dans le volume (52), et la pompe (56) est activée quand la valeur moyenne (8) est plus petite que la troisième valeur limite prédéterminée.

9. Procédé selon la revendication 7, **caractérisé en ce que** les valeurs de pression sont déterminées par détermination de la pression dans le volume (52), et la pompe (56) est activée quand la valeur moyenne (8) est plus grande que la troisième valeur limite prédéterminée.

10. Procédé selon la revendication 7, 8 ou 9, **caractérisé en ce que** la valeur moyenne (8) est une valeur moyenne glissante.
